# EUROPEAN PATENT SPECIFICATION

(11) **EP 3 438 278 B1**
(45) Date of publication and mention of the grant of the patent: **24.11.2021**
(21) Application number: 17775006.4
(22) Date of filing: 28.03.2017
(51) Int. Cl.: C12Q 1/25, C12Q 1/68, G01N 27/416, G01N 33/68

(54) **METHOD FOR QUANTIFYING AMINO ACID AND AMINO ACID QUANTIFICATION KIT**
VERFAHREN ZUR QUANTIFIZIERUNG VON AMINOSÄURE UND AMINOSÄUREQUANTIFIZIERUNGSKIT
PROCÉDÉ DE QUANTIFICATION D'ACIDES AMINÉS ET KIT DE QUANTIFICATION D'ACIDES AMINÉS

(30) Priority: 30.03.2016 JP 2016067391
(43) Date of publication of application: 06.02.2019
(73) Proprietor: Ikeda Food Research Co., Ltd., Hiroshima 721-0956 (JP); Hiroshima City University, Hiroshima-shi, Hiroshima 731-3194 (JP)
(72) Inventor: NAKATSUKA, Tomoko, Fukuyama-shi Hiroshima 721-0956 (JP); AOKI, Hideyuki, Fukuyama-shi Hiroshima 721-0956 (JP); KIDA, Mikiko, Fukuyama-shi Hiroshima 721-0956 (JP); YAMADA, Kenta, Fukuyama-shi Hiroshima 721-0956 (JP); KANEKO, Shoji, Fukuyama-shi Hiroshima 721-0956 (JP); KUGIMIYA, Akimitsu, Hiroshima-shi Hiroshima 731-3194 (JP)
(74) Representative: Dehns
(86) International application number: PCT/JP2017/012511
(87) International publication number: WO 2017/170469

(56) References cited:
- WO-A2-2004/034977
- JP-A- 2006 246 841
- JP-A- 2011 050 357
- JP-A- 2011 050 357
- JP-A- 2013 198 448
- US-A1- 2002 058 273
- US-A1- 2014 357 524
- FORBES C D ET AL: "A high-throughput competitive scintillation proximity aminoacyl-tRNA synthetase charging assay to measure amino acid concentration", ANALYTICAL BIOCHEMISTRY, ELSEVIER, AMSTERDAM, NL, vol. 363, no. 2, 15 April 2007 (2007-04-15), pages 246-254, XP025611963, ISSN: 0003-2697, DOI: 10.1016/J.AB.2007.01.027 [retrieved on 2007-03-21]

## Description

### Technical Field

The present invention relates to a method for quantifying amino acids.

### Background Art

L-amino acids (L-AA) play important roles as constituent components of proteins in living bodies and include 20 types. There have been many researches on the functionality of the L-amino acids, and the L-amino acids have been used in various industries of pharmaceuticals, processed foods, health foods and the like. For example, a free L-amino acid in a food is related to taste, scent after heating, preservability, biological control function after ingestion, etc., and has attracted attention as an important factor in fields of food science and nutritional science. Also, in recent years, it has been found that a blood level of the L-amino acid changes due to diseases, and the L-amino acid has also been utilized as a biomarker allowing diagnosis of cancers such as lung cancer, stomach cancer and colon cancer by measuring the blood level of the L-amino acid.

Also, amino acids include D-amino acids (D-AA), which are optical isomers of L-amino acids, including 19 types. Although D-amino acids are present in living bodies, their physiological functions have not been clearly understood. However, in recent years, D-amino acids have become analyzable by advancement of analytical techniques, and for example, it has been found that D-serines are increased in brains and spinal cords of Alzheimer's disease patients, that D-aspartic acids decrease in association with skin aging, and that D-alanines are involved in sweetness of crab and shrimp, etc. Thus, the physiological functions of D-amino acids have attracted attention. For this reason, the techniques for quantifying the L-form and D-form amino acids are indispensable techniques in various fields of product development, quality control, diagnosis, etc. using amino acids.

As an amino acid quantification technique for L-amino acids, a method wherein amino acids are separated by liquid chromatography and detected by color reaction with ninhydrin or a fluorescent derivatizing agent orthophthalaldehyde, is known. In addition, as amino acid quantification techniques for D-amino acids, a diastereomer method wherein D-amino acids are subjected to diastereomer fluorescence derivatization by adding a fluorescent derivatizing agent orthophthalaldehyde and a chiral derivatizing agent N-acyl-L-cysteine and analyzed by liquid chromatography, and a two-dimensional liquid chromatography method, are known (Non-Patent Document 1). However, the methods have had a problem that an analysis period is long because it takes about two hours to analyze one specimen, and the methods are unsuitable for measuring a large number of specimens.

As other amino acid quantification techniques, amino acid-measuring methods using enzymes capable of acting on L-amino acids and D-amino acids are known (Patent Document 1, Non-Patent Document 1). However, the methods have had problems that selectivity for an amino acid as a target substrate is low, the enzyme also reacts with amino acids other than the target, and there is no enzyme corresponding to 20 amino acids (19 amino acids in the case of D-amino acid).

An aminoacyl tRNA synthetase (AARS) is an enzyme related to protein synthesis in a living body and produces an aminoacyl tRNA in accordance with the following reaction formulas 1 and 2. There are 20 types of AARSs specific to 20 types of L-amino acids (L-AA). For this reason, the AARS is considered to be an enzyme with extremely high selectivity for targeted amino acids and tRNAs.

### [Formula 1]

(Reaction Formula 1) L-AA + ATP + AARS ↔ Aminoacyl AMP-AARS complex + PPi

(Reaction Formula 2) Aminoacyl AMP-AARS complex + tRNA ↔ Aminoacyl tRNA + AMP + AARS

In this reaction, pyrophosphoric acid (PPi) is produced, and at the same time, each one molecule of adenosine triphosphate (ATP) and L-amino acid (L-AA) acts on the AARS to form a reaction intermediate called an aminoacyl adenylate (aminoacyl AMP)-AARS complex. Normally, the aminoacyl AMP strongly binds to the AARS in the complex, and thus it is considered that the above-described AARS reaction does not proceed unless the complex is decomposed by adding a tRNA or a nucleophile (an amine) (Patent Document 2, Non-Patent Documents 2 to 3). In addition, it is known when a large amount of pyrophosphoric acid is produced in Reaction Formula 1 of the reaction, the aminoacyl AMP-AARS complex is decomposed by pyrophosphoric acid as a reverse reaction of Reaction Formula 1, and an ATP-PPi exchange reaction producing the amino acid, the AARS and the ATP is caused (Non-Patent Document 4).

Quantification techniques for the L-amino acid using such an AARS-related reaction (AARS reaction) have been developed so far. For example, Patent Document 3 describes an amino acid analysis method based on the reaction represented by the following Reaction Formula 3.

### [Formula 2]

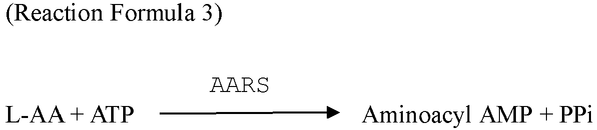

In this method, the L-amino acid is analyzed by using, as an index, pyrophosphoric acid generated while the AARS binds to the ATP and the L-amino acid. However, as can also be seen from Reaction Formula 3, since the AARS reacts with each one molecule of the L-amino acid and the ATP to produce one molecule of the aminoacyl AMP in this method, the pyrophosphoric acid is produced in only an amount equivalent to or smaller than the L-amino acid in the sample (Non-Patent Documents 5, 6, 7).

Furthermore, in Reaction Formula 3, it is expressed that the AARS functions as a catalyst and each one molecule of the ATP and the L-amino acid reacts with the AARS to produce the aminoacyl AMP and the pyrophosphoric acid, but Reaction Formula 2 does not proceed because the reaction system described in Patent Document 3 does not include the tRNA, and it is considered that the aminoacyl AMP-AARS complex is actually formed by Reaction Formula 1 (Paragraphs 0013 to 0016 in Patent Document 2). As a result, it is considered that only one molecule of the pyrophosphoric acid is produced from one molecule of the AARS. Thus, it is recognized that a large amount of AARS is required for allowing quantification of the L-amino acid contained in the sample by measuring the pyrophosphoric acid, not only from the above-described parts in Patent Document 2 but also by the inventors theirselves in Patent Document 3 (Paragraph 0046 in Patent Document 3, and Non-Patent Document 5).

As a result, since the amount of the produced pyrophosphoric acid is small relative to the L-amino acid, a quantitative range of the amino acid in the method is 300 to 900 µM by an ion-sensitive field effect transistor (ISFET) method and the amino acid can be quantified in a high concentration region, and meanwhile, in measurement of the amino acid in a low concentration region, the amino acid can be quantified in a range of 1 to 250 µM only by high sensitive analysis through fluorometry using a multistep enzymatic reaction (Non-Patent Documents 6, 7, 8).

Furthermore, the above Patent Document 2 describes an L-amino acid-quantifying method based on the AARS reaction of Reaction Formulas 1 and 2. That is, normally, the aminoacyl AMP tightly binds to the AARS to form an aminoacyl AMP-AARS complex, as described above. Thus, this method is characterized in that an amine (nucleophilic agent) such as hydroxylamine as an aminoacyl AMP-AARS complex-decomposing reagent is added so that the AARS is returned to a reactable state, and as a result, the L-amino acid is quantified with a small amount of AARS. The quantitative range of the amino acid in the method is supposed to be 5 to 200 µM.

However, in the method described in Patent Document 2, the complex-decomposing reagent reacts with the L-amino acid of the complex to produce a compound (e.g. as described in paragraph 0037 in Patent Document 2, when hydroxylamine is used as the complex-decomposing reagent, "amino acid hydroxamate" is produced), thus the L-amino acid cannot be reused, and as a result, a pyrophosphoric acid as a product is obtained only in an amount equivalent to the L-amino acid in the sample (paragraph 0023 in Patent Document 2). Furthermore, since the pyrophosphoric acid produced by the AARS reaction is detected by a multistep enzyme reaction in the method, the method is complicated and it is concerned that each enzyme is susceptible to blood contaminants and other external factors.

As described above, there have been many problems to be solved in the prior art regarding the amino acid-quantifying method capable of selectively measuring 20 types of L-amino acids and 19 types of D-amino acids, and a more effective amino acid-quantifying method is required.

On the other hand, as another AARS reaction, e.g. reactions of the following Reaction Formulas 4 and 5 are known. In this reaction, each one molecule of the ATP and the L-amino acid acts on the AARS to form the aminoacyl AMP-AARS complex. Subsequently, ATP, GTP, etc. are made to act on the complex to synthesize and produce a diadenosine polyphosphate (ApnA) such as diadenosine tetraphosphate (Ap4A), an adenosine guanosine tetraphosphate (Ap4G), and the like, which are expected as pharmaceuticals and pharmaceutical raw materials (Patent Document 4, Patent Document 5, Non-Patent Document 9, Non-Patent Document 10).

### [Formula 3]

(Reaction Formula 4) L-AA + ATP + AARS → aminoacyl AMP-AARS complex + PPi

(Reaction Formula 5) Aminoacyl AMP-AARS complex + ATP → AARS + L-AA + Ap4A

These known documents describe that the synthetic ability of the ApnA varies depending on the type of the AARS, and e.g. a tryptophan of Escherichia and an AARS of arginine do not progress the reaction of Reaction Formula 5 and cannot synthesize the Ap4A. Additionally, in Reaction Formula 4 of the reaction, a reverse reaction is promoted in the presence of a pyrophosphoric acid, and the amino acid, the AARS and the ATP are produced from the aminoacyl AMP-AARS complex. For this reason, it is necessary to prevent occurrence of the reverse reaction in order to progress the reaction, and in the techniques described in the above known documents, an inorganic pyrophosphatase capable of decomposing a pyrophosphoric acid is used in order to decompose the pyrophosphoric acid produced in Reaction Formula 4.

Furthermore, the techniques described in these known documents absolutely relate to synthesis and production of ApnA, Ap4G, etc. and are not intended to solve technical problems related to quantification of amino acids. Actually, in these known documents, there is no description on the quantification of amino acids utilizing the AARS reaction of the above Reaction Formulas 4 and 5, and no description on the reuse of the amino acid and the AARS caused in Reaction Formula 5.

AARS is known to include 20 types of AARSs specific to 20 types of L-amino acids. There is a report regarding some AARSs, wherein this AARS acts on D-amino acids (Non Patent Document 11). However, this document relates to an aspect that the AARS acts on the D-amino acid and transfers it to the tRNA, but is not intended to solve technical problems regarding the quantification of D-amino acids. In fact, in the document, there is no description on the quantification of D-amino acids utilizing the AARS reactions of the above Reaction Formulas 4 and 5, and no description on reuse of the D-amino acid and the AARS caused in Reaction Formula 5.

### Prior Art Documents

### Patent Documents

Patent Document 1: Japanese Patent Application Laid-Open No. 2013-146264
Patent Document 2: JP Pat. No. 5305208
Patent Document 3: Japanese Patent Application Laid-Open No. 2011-50357
Patent Document 4: Japanese Patent Application Laid-Open No. 2000-69990
Patent Document 5: JP Pat. No. 3135649

### Non-Patent Documents

Non-Patent Document 1: "Kagaku to Seibutsu (Chemistry and Organisms)", Vol. 53, p. 192-197, Japan, 2015
Non-Patent Document 2: "Voet's Biochemistry (Second volume)", Japan, 3rd edition, 1024-1029
Non-Patent Document 3: J. Biol. Chem., 241, 839-845, 1996 Non-Patent Document 4: "Journal of the Crystallographic Society of Japan", Vol. 52, p. 125-132, Japan, 2010
Non-Patent Document 5: Web page of Hiroshima City University [searched on January 13, 2016], Internet (URL: http://rsw.office.hiroshimacu.ac.jp/Profiles/2/000129/profile.html) [online], Akimitsu Kugimiya, majoring in creative science, Faculty of Information Sciences, Hiroshima City University
Non-Patent Document 6: Analytical Biochem., 443, 22-26, 2013
Non-Patent Document 7: Appl. Biochem. Biotechnol., 174, 2527-2536, 2014
Non-Patent Document 8: J. Chem. Eng. 6, 397-400, 2012
Non-Patent Document 9: "Journal of Tokyo Medical University", Vol. 51, p. 469-480, Japan, 1993
Non-Patent Document 10: Agric. Biol. Chem., 53, 615-623, 1989
Non-Patent Document 11: Biosci, Biotechnol. Biochem., 69, 1040-1041, 2005

### Summary of Invention

### Problem to be solved

The objects of the present invention are to solve various problems in the above-described prior art regarding the method for quantifying L-form and D-form amino acids, and to provide a method for selectively and easily quantifying the L-form and/or D-form amino acids to be measured using an AARS with high sensitivity.

### Solution to Problem

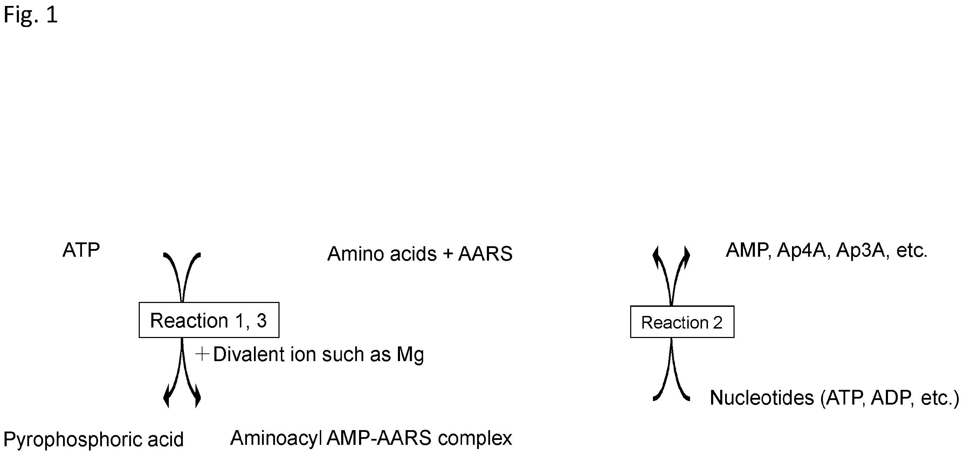

As a result of various investigations, the inventors have found that, in a method for quantifying amino acids in a sample using an AARS, an aminoacyl tRNA synthetase (AARS) and amino acids (L-AA and/or D-AA) are released from an aminoacyl AMP-AARS complex once formed as shown in Figure 1, and they are used again for forming the aminoacyl AMP-AARS complex, so that reaction products such as pyrophosphoric acid to be measured can be ultimately produced up to a molar number larger than that of the amino acids contained in the sample, and this finding has led to the completion of the invention.

The present invention relates to the following aspects of [1] to [7].
[1] A method for quantifying amino acids in a sample, which includes Step (I) including the following steps:
   (Step 1-1) a step including a reaction (Reaction 1) wherein L-form and/or D-form amino acids (L-AA and/or D-AA) in the sample, an aminoacyl tRNA synthetase (AARS) corresponding to the amino acids and an adenosine triphosphate (ATP) are reacted in the presence of a divalent ion or a polyamine to form a complex comprising an aminoacyl adenylate (aminoacyl AMP) and the AARS (aminoacyl AMP-AARS complex);
   (Step 1-2) a step including a reaction (Reaction 2) wherein the aminoacyl AMP-AARS complex formed in Reaction 1 and/or Reaction 3 is reacted with a nucleotide to release the AARS and the amino acids (L-AA and/or D-AA) from the complex;
   (Step 1-3) a step including a reaction (Reaction 3) wherein the amino acids (L-AA and/or D-AA) and/or the AARS released in Reaction 2 are reused in Reaction 1 to cause the aminoacyl AMP-AARS complex reaction; and
   (Step 1-4) a step of repeating Step 1-2 and Step 1-3, and
   Step (II) including measuring an amount of reaction products produced in Step (I), wherein the molar number of the reaction products produced in Step (I) is larger than that of the amino acids in the sample, and determining an amount of the L-form and/or D-form amino acids on the basis of the measured amount of the reaction products.
[2] The method for quantifying amino acids according to [1], wherein the amount of the reaction products produced in Step (I) is measured by measuring potential change by an ion-sensitive field effect transistor, a glass electrode or a multielectrode electrometer.
[3] The method for quantifying amino acids according to [1], wherein the amount of the reaction products produced in Step (I) is measured by measuring change in absorbance in accordance with absorptiometry.
[4] The method for quantifying amino acids according to any one of [1] to [3], wherein at least one of a pyrophosphoric acid and hydrogen ion is measured as the reaction products produced in Step (I).
[5] The method for quantifying amino acids according to any one of [1] to [4], further comprising a pre-treatment step for removing either one of the L-form and D-form amino acids in the sample.

### Effects of Invention

In the amino acid-quantifying method according to the present invention, an AARS and amino acids (L-form and/or D-form amino acids) are released from a formed aminoacyl AMP-AARS complex, and they are repeatedly used for forming the aminoacyl AMP-AARS complex, so that reaction products such as pyrophosphoric acid to be measured can be produced up to a molar number larger than that of the amino acids contained in the sample. As a result, even when these reaction products are measured by a means simpler than the prior art, amino acids can be quantified in a range equivalent to the amino acid quantification range in the amino acid-quantifying method which is high sensitive analysis by fluorometry or the like using a multistep enzyme reaction in the prior art, and thus such a high sensitive analysis is not required. In addition, when the same measuring means is used, amino acids can be quantified in a lower concentration range. Furthermore, both the L-form and D-form amino acids can be quantified. Additionally, after removing either one of the L-form and D-form amino acids, the remaining amino acid can also be measured by the AARS.

Consequently, the present invention can provide a method for selectively and easily quantifying amino acids to be measured using an AARS with high sensitivity.

### Brief Description of Drawings

Figure 1 illustrates reaction steps of the present invention.
Figure 2 illustrates yields of pyrophosphoric acids depending on concentrations of various AARSs using L-amino acids.
Figure 3 illustrates yields of pyrophosphoric acids depending on various ATP concentrations in various AARSs using L-amino acids.
Figure 4-1 illustrates yields of pyrophosphoric acids depending on various divalent ions in various AARSs using L-amino acids.
Figure 4-2 illustrates yields of pyrophosphoric acids depending on various divalent ions in various AARSs using L-amino acids.
Figure 5-1 illustrates yields of pyrophosphoric acids depending on various nucleotides in various AARSs using L-amino acids.
Figure 5-2 illustrates yields of pyrophosphoric acids depending on various nucleotides in various AARSs using L-amino acids.
Figure 6 illustrates yields of pyrophosphoric acids in the AARS reactions according to the present invention and the known documents (Non-Patent Documents 6 and 7).
Figure 7 illustrates comparison of yields of pyrophosphoric acids in the AARS reaction between the presence and the absence of nucleophilic agent.
Figure 8 illustrates temperature dependency of the AARS.
Figure 9 illustrates calibration curves for L-amino acids in pyrophosphoric acid measurement in accordance with a molybdenum blue method.
Figure 10 illustrates calibration curves for L-amino acids in hydrogen ion concentration measurement by a cumulative ISFET electrode.
Figure 11 illustrates yields of pyrophosphoric acids depending on various ATP concentrations and divalent ions in various AARSs using D-amino acids.
Figure 12 illustrates calibration curves for D-amino acids in pyrophosphoric acid measurement in accordance with a molybdenum blue method.
Figure 13 illustrates measurement of D-amino acids after removal of L-amino acids.

### Description of Embodiments

In Reactions 1 and 3 in the method of the present invention, the amino acids, the AARS corresponding to the amino acids, and the ATP are reacted to form the aminoacyl AMP-AARS complex. For the AARS used in the method of the present invention, an AARS which specifically acts on 20 types of amino acids is used. For example, in the case of histidine (His), an AARS which specifically acts on histidine (HisRS) is used, in the case of serine (Ser), an AARS which specifically acts on serine (SerRS), in the case of tryptophan (Trp), an AARS which specifically acts on tryptophan (TrpRS) is used, and so on. In addition, the AARS used in the present invention may be any AARS as long as it is an AARS derived from an organism like an animal such as cattle, rat and mouse, a plant such as Lupin seed and Phaseolus aurus, and a microorganism such as Escherichia, Thermus, Thermotoga and Saccharomyces. Above all, the microorganism-derived AARS is preferred from the viewpoints of handling and productivity. Also, it may be a recombinant AARS or a synthesized AARS. Although it is preferably a soluble enzyme, it may be an insoluble enzyme combined with a surfactant, and may be an enzyme obtained by solubilizing an insoluble enzyme by fusion with a solubilized protein, deletion of a membrane-bound portion, or the like. The known amino acid sequence of the AARS can be used, and for the recombinant AARS, a protein having a sequence identity of 60%, 65%, 70%, 75%, 80%, 85%, 90%, 95% or higher and having an AARS activity may be used.

As the AARS prepared and used in the present invention, an AARS or the like obtained by any method/means known to those skilled in the art can be used, wherein e.g. an object containing the AARS is hydrogenated, pulverized by a pulverizer, an ultrasonic pulverizer or the like, then, from the pulverized product, solid matters are removed by centrifugation, filtration or the like to obtain an extract, and furthermore the extract is purified and isolated by column chromatography or the like. That is, a main technical feature of the present invention is that the AARS and the amino acids are released from the formed aminoacyl AMP-AARS complex, they are repeatedly used for forming the aminoacyl AMP-AARS complex, so that the reaction products such as a pyrophosphoric acid to be measured are produced up to a molar number larger than that of the amino acids contained in the sample, in the amino acid-quantifying method using the AARS. The method for preparing the AARS is not limited at all.

The sample used in the present invention is not particularly limited, and examples thereof include blood, fresh food, processed food and beverages. The amino acid concentration in each sample varies depending on each amino acid. For example, in relation to blood levels of the amino acids, the blood contains 11 to 44 nmol/mL of glutamic acid, 19 to 33 nmol/mL of methionine, 41 to 66 nmol/mL of tryptophan, 50 to 83 nmol/mL of tyrosine, 92 to 162 nmol/mL of serine, 68 to 97 nmol/mL of histidine, 119 to 257 nmol/ml of lysine, 488 to 733 nmol/ml of glutamine, 240 to 510 nmol/mL of alanine, and the like. In relation to contents of free amino acids in a fresh food, for example, a garlic contains 5 mg/100 g of lysine, 136 mg/100 g of arginine, 6 mg/100 g of serine, and the like. A tomato contains 94 mg/100 g of glutamine, 106 mg/100 g of proline, and the like. A dried shiitake mushroom contains 386 mg/100 g of glutamic acid, 268 mg/100 g of threonine, 46 mg/100 g of serine, and the like. Additionally, in relation to contents of L-form and D-form amino acids in a fruit, an apple contains 2071 µmol/L of L-form asparagine, 15 µmol/L of D-form asparagine, 105 µmol/L of L-form alanine and 3 µmol/L of D-form alanine, and a pineapple contains 3109 µmol/L of L-form asparagine, 25 µmol/L of D-form asparagine, 202 µmol/L of L-form valine, 2 µmol/L of D-form valine, and the like. In relation to contents of free amino acids in a processed food or a beverage, for example, Soy sauce contains 213 mg/100 g of lysine, 104 mg/100 g of histidine, 782 mg/100 g of glutamic acid, and the like. Green tea contains 107 mg/100 g of serine, 314 mg/100 g of arginine, 258 mg/100 g of glutamic acid, and the like. A fully ripened green robusta coffee bean contains 10 mg/100 g of lysine, 48 mg/100 g of histidine, 43 mg/100 g of serine, and the like. The sample is appropriately diluted and prepared depending on the expected concentrations of the amino acids in each of these samples, so that the sample can be used as the sample of the present invention.

The amino acids in the sample used in the present invention may include both L-form and D-form amino acids. In such a case, for example, it is preferred that either one of the L-form and D-form amino acids is removed as pretreatment in the method of the present invention. The method for removing either one of L-form and D-form amino acids can be exemplified by any method known to those skilled in the art, such as a method for removing either one of the L-form and D-form amino acids by column chromatography or an appropriate enzyme. Note that, although a biological sample contains both L-form and D-form amino acids, a ratio of most D-form amino acids in a mammalian body is about 0.1 to 1.0% of the L-form amino acids, and a ratio of the D-form amino acids in a fruit is 3.0% or less of the L-form amino acids, and thus the biological sample does not contain D-form amino acids so much as to affect the quantification of the L-form amino acids.

An AARS concentration in the reaction solution used for the reaction can be appropriately determined by a person skilled in the art depending on various reaction conditions such as the type of the sample, the estimated concentration of the amino acids in the sample, the ATP concentration and the reaction time/temperature. In order to suppress the reverse reaction in Reaction 1 of Step (I) as much as possible, it is preferred that the AARS concentration is increased when the amino acid concentration in the sample is expected to be low, and conversely, a low AARS concentration is allowed when the amino acid concentration in the sample is expected to be high. In addition, the reaction can be completed in a short time by increasing the AARS concentration, and conversely, when a long reaction time is allowed, a low AARS concentration is allowed. For example, a concentration of AARS derived from a microorganism such as Escherichia, Thermus and Thermotoga may be 0.05 µM or higher, more preferably 0.1 µM or higher, further preferably 0.5 µM or higher, particularly preferably 1.0 µM or higher, and most preferably 5.0 µM or higher. In any case, since the AARS is repeatedly used in the method of the present invention, the method has an advantage that there is no need to add an excessive amount of AARS to an expected amount of amino acid in the sample. Thus, the upper limit of the AARS concentration can be appropriately set by a person skilled in the art in consideration of economic efficiency and the like.

In Reaction 1 or Reaction 3 in the method of the present invention, ATP and divalent ions are used together with the AARS. For the ATP used in the present invention, a sodium salt, a lithium salt and the like can be used. The concentration of the ATP in the reaction solution used for the reaction can be appropriately determined by a person skilled in the art depending on various reaction conditions such as the type of the sample, the estimated amino acid concentration in the sample, the AARS concentration, the nucleotide concentration and the reaction time/temperature, but preferably the ATP is added in an amount excessive relative to the estimated amino acid concentration in the sample. For example, when the sample is blood, the ATP concentration may be 0.05 mM or higher, more preferably 0.1 mM or higher, further preferably 1.0 mM or higher, particularly preferably 5.0 mM or higher, most preferably 10.0 mM or higher. The upper limit of the ATP concentration can be appropriately set by a person skilled in the art in consideration of economic efficiency and the like. For the divalent ions used in the present invention, magnesium, manganese, cobalt, calcium and the like can be used. Since divalent ions have different requirements depending on the AARS, divalent ions suitable for the AARS to be used should be appropriately used, but it is more preferred to use magnesium or manganese which have common requirements for the AARS. Furthermore, polyamines such as spermine, spermidine and putrescine which have the same actions as of divalent ions can also be used. Although the concentration of the divalent ions in the reaction solution used for the reaction can be appropriately determined, it is preferred that the divalent ions are add in an amount equivalent to or larger than the ATP concentration. For example, a ratio of ATP:divalent ions in the AARS derived from a microorganism such as Escherichia, Thermus and Thermotoga may be at least 1:1, more preferably at least 1:3, even more preferably at least 1:5, particularly preferably at least 1:7, most preferably at least 1:10.

Subsequently, in Reaction 2 in the method of the present invention, the aminoacyl AMP-AARS complex formed in Reaction 1 and/or Reaction 3 is reacted with a nucleotide to decompose the complex, and the AARS and the amino acids are released from the complex. As the nucleotide used for the reaction, one arbitrarily selected from ATP, adenosine diphosphate (ADP), adenosine monophosphate (AMP), guanosine triphosphate (GTP), deoxyadenosine triphosphate (dATP) and the like, or a combination thereof can be used. In Reaction 2, AMP, Ap4A, Ap3A, Ap4G, Ap3G, and the like are produced depending on the nucleotide to be used, at the same time of release of the AARS and the amino acids from the aminoacyl AMP-AARS complex. The concentration of the nucleotide in the reaction solution used for the reaction can be appropriately determined by a person skilled in the art depending on various reaction conditions such as the type of the sample, the estimated amino acid concentration in the sample, the AARS concentration, the ATP concentration and the reaction time/temperature, but preferably the nucleotide is added in an amount excessive relative to the amino acid concentration in the sample, because the nucleotide is consumed in production of AMP, Ap4A, Ap3A, Ap4G, Ap3G and the like. For example, when the sample is blood, the nucleotide concentration may be 0.05 mM or higher, more preferably 0.1 mM or higher, further preferably 1.0 mM or higher, particularly preferably 5.0 mM or higher, most preferably 10.0 mM or higher. Thus, in the method of the present invention, addition of the aminoacyl AMP-AARS complex-decomposing reagent such as an amine as described in Patent Document 2 is not required for returning the AARS to the reactable state, and since the released amino acid does not react with the reagent, the released amino acid can be reused for forming the aminoacyl AMP-AARS complex.

In Reaction 3 in the method of the present invention, the amino acids and/or the AARS released in Reaction 2 is reused in Reaction 1, to cause the aminoacyl AMP-AARS complex reaction. Furthermore, in Step (I) in the method of the present invention, the ATP produced by reacting the pyrophosphoric acid produced in Reactions 1 and 3 and the AMP in the reaction system with phosphoenolpyruvic acid and pyruvate dikinase can be reused for formation of the aminoacyl AMP-AARS complex and release of the AARS and the amino acids from the complex, and/or an ADP produced by making the Ap4A pyrophosphohydrolase act on an Ap4A produced from the nucleotide in Reaction 2 can be reused as the nucleotide in Reaction 2, by any method known to those skilled in the art.

As a result, under reaction conditions such as compositions of the nucleotide such as ATP and the AARS corresponding to the amino acids described above, molecules of each reaction product such as the pyrophosphoric acid and/or the hydrogen ions can be produced in a molar number larger than that of the amino acids to be measured in the sample, as a result of the reaction in Step (I). As a result, the amino acids can be quantified from an extremely low amino acid concentration of about 1 µM, which is lower than the concentration in the prior art, in the method of the present invention. Hence, it can be said that this point is a remarkable effect of the present invention as compared with the prior art.

However, even if the amount of the reaction products such as pyrophosphoric acid produced under the reaction conditions is not larger than the molar number of the amino acids in the sample, the reaction products such as pyrophosphoric acid need not be produced in a molar number larger than that of the amino acids as long as the amino acids can be quantified on the basis of the reaction products. Thus, the amounts of pyrophosphoric acid and hydrogen ions produced in Step (I) of the present invention are not particularly limited as long as the amino acids can be quantified by an appropriate method for measuring the reaction products in Step (II). Also, the repeat count of Reaction 2 (Step 1-2) and Reaction 3 (Step 1-3) in (Step 1-4) in the method of the present invention is not particularly limited as long as the amino acid can be quantified by an appropriate method for measuring the reaction products in Step (II).

The reaction temperature in Step (I) in the method of the present invention may be any temperature for causing each reaction. For example, in the case of the AARS of Escherichia, the temperature is preferably 10 to 80°C, more preferably 20 to 65°C, most preferably 30 to 60°C. In the cases of the AARSs of Thermus and Thermotoga, the temperature is preferably 10 to 100°C, more preferably 30 to 98°C, most preferably 50 to 95°C. Also, the reaction time may be any period for causing an AARS reaction with the amino acids in the sample, but it is preferably about 1 to 90 minutes, more preferably about 5 to 60 minutes, further preferably about 10 to 30 minutes. Furthermore, the reaction pH may also be any pH for causing an AARS reaction with the amino acids in the sample. For example, in the cases of the AARSs of Escherichia, Thermus and Thermotoga, the pH is preferably 4.0 to 10.0, more preferably 5.0 to 9.8, most preferably 6.0 to 9.5. Note that the reaction pH can be adjusted using any buffer or the like known to those skilled in the art.

Respective reaction components such as reagents and enzymes used in each step included in Step (I) in the method of the present invention can be added to the reaction system in accordance with any means, procedure and the like known to those skilled in the art, as long as the addition method can cause the AARS reaction. For example, respective components may be previously added to the reaction system at a time before starting the reaction, or alternatively the AARS or the amino acids may be finally added for reaction.

In Step (II) in the method of the present invention, each amount of each reaction product such as pyrophosphoric acid, adenosine monophosphate (AMP) and hydrogen ion produced in Step (I) is measured, where the molar number of the reaction products produced in Step (I) is larger than that of the amino acids in the sample, to determine an amount of the amino acid on the basis of the measured amount of the reaction product. Depending on the measurement method or the like, Step (II) can be appropriately carried out after the AARS reaction with the amino acids in the sample in Step (I) is terminated by any method/means known to those skilled in the art such as addition of trichloroacetic acid to the reaction system e.g. as described in Examples, or at any stage where the reaction progresses in Step (I).

For measuring the amount of pyrophosphoric acid produced in Step (I) of the present invention, any method/means known to those skilled in the art, e.g. enzyme methods capable of measuring pyrophosphoric acid, such as a molybdenum blue method wherein an absorbance of a blue complex produced by reacting molybdic acid with pyrophosphoric acid is measured, a method of combining a hypoxanthine-guanine phosphoribosyl transferase and a xanthine oxidase or a xanthine dehydrogenase, a method of measuring luminescence of a combined product of luminol with an inorganic pyrophosphatase, pyruvate oxidase and peroxidase, or the like can be used. Also, a measurement method of measuring a potential change by a multielectrode electrometer, wherein an oxidation-reduction reaction is caused by an enzyme reaction or the like, and a current value attributed to the oxidation-reduction reaction is detected, or the like can be used. Furthermore, for measuring hydrogen ions produced in the reaction, a measurement method of measuring a potential change by a glass electrode for detecting hydrogen ions or an ion-sensitive field effect transistor, or the like can be used. For measuring the adenosine monophosphate (AMP) produced by the reaction, measurement with an AMP sensor utilizing luminescence for detecting the adenosine monophosphate, or the like can be used. The pyrophosphoric acid, hydrogen ions, AMP, etc. produced in Step (I) of the present invention can be separated from the reaction solution and measured. Although the method for separating the pyrophosphoric acid, hydrogen ions, AMP, etc. from the reaction solution is not particularly limited as long as the method does not affect the measurement, examples of the method include protein removal by acid treatment, paper chromatography separation, separation by a microfluidic device, and the like. A main technical feature of the present invention is that, in the amino acid-quantifying method using AARS, the AARS and the amino acids are released from the formed aminoacyl AMP-AARS complex, and these compounds are repeatedly reused for formation of the complex to produce the reaction products such as pyrophosphoric acid to be measured up to a molar number larger than that of the amino acids contained in the sample. The method for measuring an amount of the reaction products is not limited at all.

The present specification describes also an amino acid quantification kit for performing the above-described method of the present invention, which includes each component described above required for quantifying the amino acids in the sample. The kit may appropriately contain other optional components known to those skilled in the art such as a stabilizer and a buffer to enhance the stability of the reagent components such as an enzyme. Although the components are not particularly limited as long as they do not affect the measurement, they can be exemplified by bovine serum albumin (BSA), an antioxidant, a surfactant, or amino acids having no activity with an enzyme, and the like. In addition, an example of the kit is a kit for measuring the above-described pyrophosphoric acid and hydrogen ions.

### Examples

Hereinafter, the present invention will be specifically explained with reference to Examples, but the technical scope of the present invention is not limited by the following Examples.

### (Preparation of AARS)

### [Example 1]

An E. coli BL21 (DE3) pLys strain was transformed with a plasmid (pET28b) having AARS sequences derived from Thermus and Thermotoga, and this was used as an expression strain. Each expression strain was cultured in a TB medium containing kanamycin and chloramphenicol as selection markers at 37°C, and after the OD600 reached about 0.6, IPTG was added so that its final concentration was 1 mM, and induction culture was carried out at 25°C overnight. After completion of the culture, the bacteria were collected, and the obtained bacteria were crushed by sonication to prepare a cell-free extract. The prepared cell-free extract was subjected to heat treatment at 70°C for 15 minutes and then centrifuged. The expression of the desired enzyme was confirmed by electrophoresis using a portion of the obtained supernatant. Subsequently, from the remaining supernatant, contaminant proteins were removed by a His tag column (trade name: TALON superflow, made by GE Healthcare) to obtain HisRS, SerRS, TrpRS and LysRS.

### [Example 2]

An E. coli BL21 (DE3) pLys strain was transformed with a plasmid (pET28b) having an AARS sequence derived from E. coli K12, and used as an expression strain. Each expression strain was cultured in a TB medium containing kanamycin and chloramphenicol as selection markers at 37°C, and after the OD600 reached about 0.6, IPTG was added so that its final concentration was 1 mM, and induction culture was carried out at 25°C overnight. After completion of the culture, the bacteria were collected, and the obtained bacteria were crushed by sonication to prepare a cell-free extract. Furthermore, the extract was centrifuged, and the expression of the desired enzyme was confirmed by electrophoresis using a portion of the obtained supernatant. Subsequently, from the remaining supernatant, contaminant proteins were removed by a His tag column (trade name: TALON superflow, made by GE Healthcare) to obtain TyrRS, HisRS, SerRS and TrpRS.

### (Yield of pyrophosphoric acid depending on concentrations of various AARSs using L-amino acids: Step (I) in the method of the present invention)

### [Example 3]

240 µL of a reaction solution containing 250 mM HEPES-KOH (pH 8), 31.3 mM ATP and 313 mM MgCl₂ was prepared, to which 30 µL of L-histidine was added so that its final concentration was 30 µM, and 30µL of HisRS (derived from thermophile) was added so that its final concentration was 0.1 µM, and the solution was treated at 70°C for 30 minutes. After the reaction, 60 µL of trichloroacetic acid was added so that its final concentration was 4%, to terminate the reaction. After the termination of the reaction, the precipitate was removed by centrifugation to prepare Example Product (inventive product) 1.

### [Example 4]

240 µL of a reaction solution containing 250 mM HEPES-KOH (pH 8), 31.3 mM ATP and 313 mM MgCl₂ was prepared, to which 30 µL of L-histidine was added so that its final concentration was 30 µM, and 30 µL of HisRS (derived from E. coli) was added so that its final concentration was 0.12 µM, 0.17µM or 0.21 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 2, 3 and 4.

### [Example 5]

240 µL of a reaction solution containing 250 mM HEPES-KOH (pH 8), 31.3 mM ATP and 313 mM MgCl₂ was prepared, to which 30 µL of L-serine was added so that its final concentration was 30 µM, and 30µL of SerRS (derived from thermophile) was added so that its final concentration was 0.05 µM, 0.075 µM or 0.1 µM, and the solution was treated at 70°C for 30 minutes. After the reaction, 60 µL of trichloroacetic acid was added so that its final concentration was 4%, to terminate the reaction. After the termination of the reaction, the precipitate was removed by centrifugation to prepare Example Products 5, 6 and 7.

### [Example 6]

240 µL of a reaction solution containing 250 mM HEPES-KOH (pH 8), 31.3 mM ATP and 313 mM MgCl₂ was prepared, to which 30 µL of L-serine was added so that its final concentration was 30 µM, and 30 µL of SerRS (derived from E. coli) was added so that its final concentration was 0.12 µM, 0.17 µM or 0.21 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 8, 9 and 10.

### (Measurement of pyrophosphoric acid in accordance with molybdenum blue method: Step (II) in the method of the present invention)

### [Example 7]

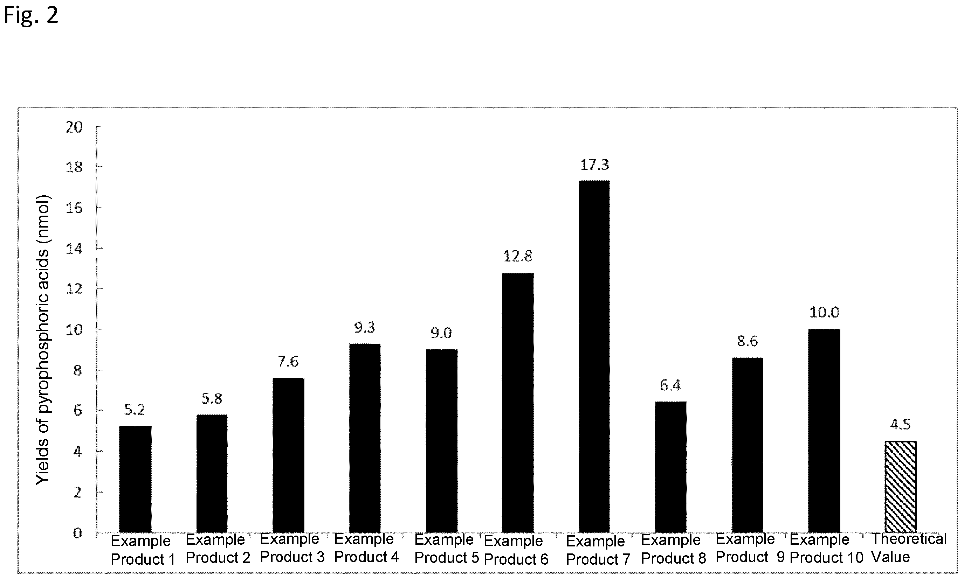

15 µL of 1 M mercaptoethanol and 60 µL of color developing liquid (2.5% ammonium molybdate/5 N sulfuric acid) were mixed in 150 µL of a reaction solution of each of the prepared Example Products 1 to 10, the solution was allowed to stand at room temperature for 20 minutes, and then an absorbance at 580 nm was measured. The pyrophosphoric acid amount in the reaction solution was determined from a value obtained by subtracting the absorbance value of each sample to which water was added instead of the L-amino acid as a blank from the absorbance value of the respective sample. As a result, as shown in Figure 2, the pyrophosphoric acid was produced in an amount larger than the theoretical value of the pyrophosphoric acid amount produced when all of the added amino acids were used in the enzyme reaction. Thus, it was revealed that the pyrophosphoric acid could be produced in a molar number larger than the molecular number of the amino acids contained in the sample in accordance with the method of the present invention.

### (Yield of pyrophosphoric acid depending on concentrations of various ATPs using L-amino acids: Step (I) in the method of the present invention)

### [Example 8]

240 µL of a reaction solution containing 250 mM HEPES-KOH (pH 8), 6.3 mM ATP and 63.5 mM MgCl₂, as well as 240 µL of a reaction solution containing 250 mM HEPES-KOH (pH 8), 31.3 mM ATP and 313 mM MgCl₂ were prepared, and to each of the solutions, 30 µL of L-histidine was added so that its final concentration was 30 µM, and 30 µL of HisRS (derived from thermophile) was added so that its final concentration was 5 µM, and the solution was treated at 70°C for 15 minutes. After the reaction, 60 µL of trichloroacetic acid was added so that its final concentration was 4%, to terminate the reaction. After the termination of the reaction, the precipitate was removed by centrifugation to prepare Example Products 11 and 12.

### [Example 9]

240 µL of a reaction solution containing 250 mM HEPES-KOH (pH 8), 12.5 mM ATP and 125 mM MgCl₂, as well as 240 µL of a reaction solution containing 250 mM HEPES-KOH (pH 8), 31.3 mM ATP and 313 mM MgCl₂ were prepared, and to each of the solutions, 30 µL of L-tyrosine was added so that its final concentration was 30 µM, and 30 µL of TyrRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 13 and 14.

### (Measurement of pyrophosphoric acid in accordance with molybdenum blue method: Step (II) in the method of the present invention)

### [Example 10]

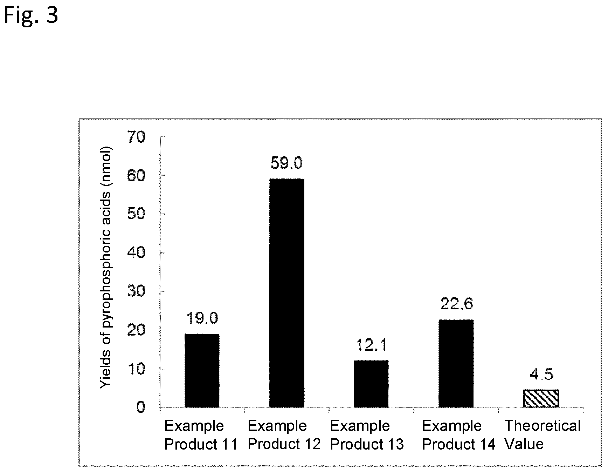

The prepared pyrophosphoric acids of Example Products 11 to 14 were measured in accordance with the molybdenum blue method described in Example 7. As a result, there was a tendency that the pyrophosphoric acid increased as the ATP concentration increased, as shown in Figure 3. In addition, the pyrophosphoric acid was produced in an amount larger than the theoretical value of the pyrophosphoric acid amount produced when all of the added amino acids were used in the enzyme reaction. Thus, it was shown that the pyrophosphoric acid could be produced in a molar number larger than the molecular number of the amino acids contained in the sample in accordance with the method of the present invention.

### (Yield of pyrophosphoric acid depending on various divalent ions in various AARSs using L-amino acids: Step (I) in the method of the present invention)

### [Example 11]

240 µL of reaction solutions respectively containing 250 mM HEPES-KOH (pH 8), 31.3 mM ATP and 31.3 mM MgCl₂ or MnCl₂ or CoCl₂ were prepared, and to each solution, 30 µL of L-serine was added so that its final concentration was 30 µM, and 30µL of SerRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 15 to 17.

### [Example 12]

240 µL of reaction solutions containing 250 mM HEPES-KOH (pH 8), 31.3 mM ATP and 31.3 mM MgCl₂ or MnCl₂ or CoCl₂ were prepared, and to each solution, 30 µL of L-tyrosine was added so that its final concentration was 30 µM, and 30 µL of TyrRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 18 to 20.

### [Example 13]

240 µL of reaction solutions containing 250 mM HEPES-KOH (pH 8), 31.3 mM ATP and 31.3 mM MgCl₂ or MnCl₂ or CoCl₂ were prepared, and to each solution, 30 µL of L-histidine was added so that its final concentration was 30 µM, and 30 µL of HisRS (derived from thermophile) was added so that its final concentration was 5 µM, and the solution was treated at 70°C for 30 minutes. After the reaction, 60 µL of trichloroacetic acid was added so that its final concentration was 4%, to terminate the reaction. After the termination of the reaction, the precipitate was removed by centrifugation to prepare Example Products 21 to 23.

### [Example 14]

240 µL of reaction solutions containing 250 mM HEPES-KOH (pH 8), 31.3 mM ATP and 31.3 mM MgCl₂ or MnCl₂ or CoCl₂ were prepared, and to each solution, 30 µL of L-serine was added so that its final concentration was 30 µM, and 30 µL of SerRS (derived from thermophile) was added so that its final concentration was 5 µM, and the solution was treated at 70°C for 30 minutes. After the reaction, 60 µL of trichloroacetic acid was added so that its final concentration was 4%, to terminate the reaction. After the termination of the reaction, the precipitate was removed by centrifugation to prepare Example Products 24 to 26.

### (Measurement of pyrophosphoric acid in accordance with molybdenum blue method: Step (II) in the method of the present invention)

### [Example 15]

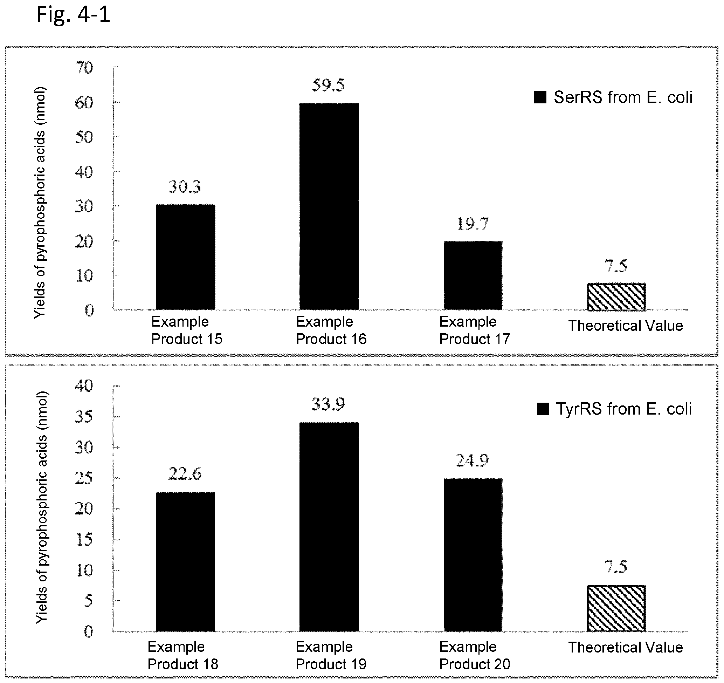

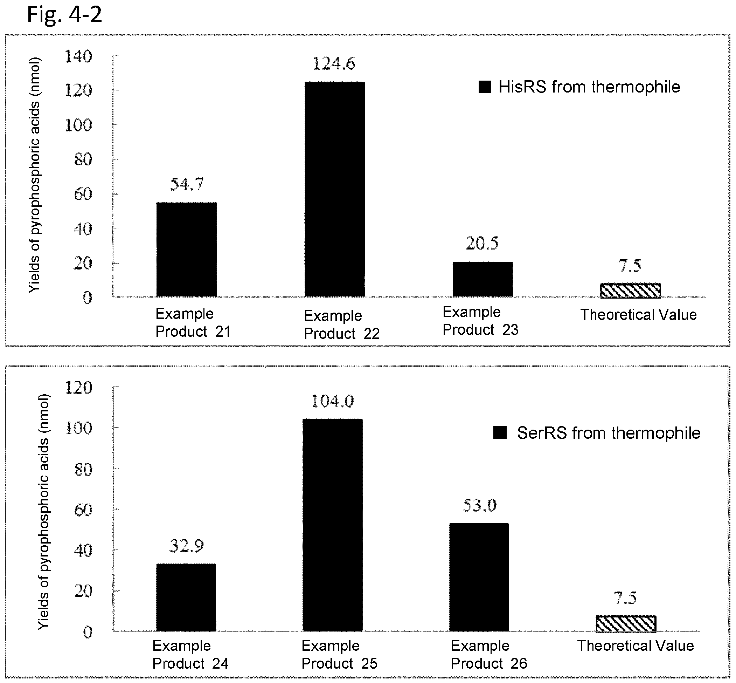

The Example Products 15 to 26 prepared in Examples 11 to 14 were measured in accordance with the molybdenum blue method described in Example 7. The result indicated that, among the same AARSs, the yield of the pyrophosphoric acid varied depending on the type of the bivalent cation, as shown in Figure 4. In addition, it was indicated that although the influence of the divalent ion on the yield of the pyrophosphoric acid also varied depending on the type of the AARS, Mg and Mn were optimum divalent ions commonly having for the AARSs.

### (Yield of pyrophosphoric acid depending on various nucleotides in various AARSs using L-amino acids: Step (I) in the method of the present invention)

### [Example 16]

240 µL of a reaction solution containing 250 mM HEPES-KOH (pH 8), 31.3 mM ATP and 31.3 mM MgCl₂, or 240 µL of a reaction solution containing 250 mM HEPES-KOH (pH 8), 31.3 mM ATP, 31.3 mM ADP and 313 mM MgCl₂, or 240 µL of a reaction solution containing 250 mM HEPES-KOH (pH 8), 31.3 mM ATP, 31.3 mM AMP and 313 mM MgCl₂ was prepared, and to each solution, 30 µL of L-tryptophan was added so that its final concentration was 50 µM, and 30µL of TrpRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 27 to 29.

### [Example 17]

240 µL of a reaction solution containing 250 mM HEPES-KOH (pH 8), 31.3 mM ATP and 313 mM MgCl₂, or 240 µL of a reaction solution containing 250 mM HEPES-KOH (pH 8), 31.3 mM ATP, 31.3 mM ADP and 313 mM MgCl₂ was prepared, and to each solutions, 30 µL of L-histidine was added so that its final concentration was 50 µM, and 30 µL of HisRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 30 and 31.

### [Example 18]

240 µL of a reaction solution containing 250 mM HEPES-KOH (pH 8), 31.3 mM ATP and 31.3 mM MgCl₂, or 240 µL of a reaction solution containing 250 mM HEPES-KOH (pH 8), 31.3 mM ATP, 31.3 mM AMP and 313 mM MgCl₂ was prepared, and to each solutions, 30 µL of L-tyrosine was added so that its final concentration was 50 µM, and 30 µL of TyrRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 32 and 33.

### (Measurement of pyrophosphoric acid in accordance with molybdenum blue method: Step (II) in the method of the present invention)

### [Example 19]

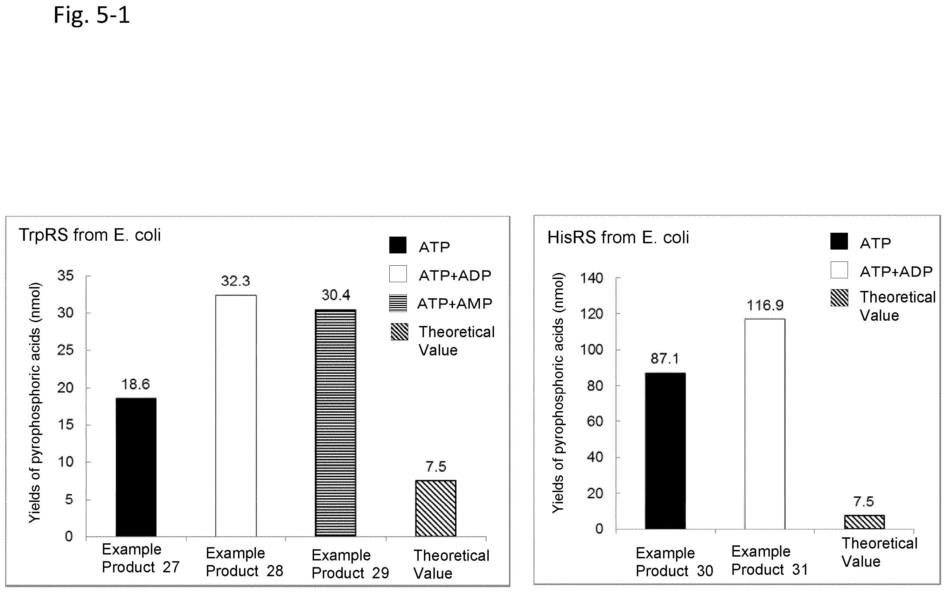

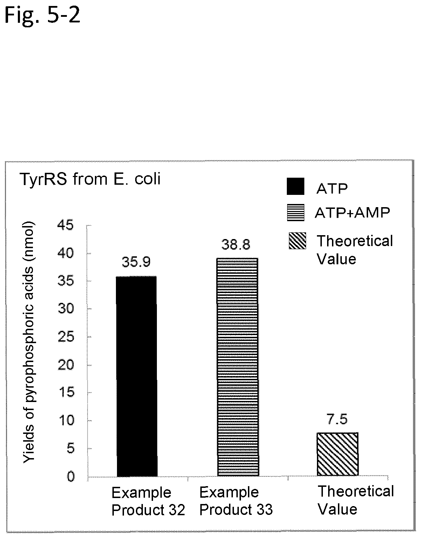

The Example Products 27 to 33 prepared in Examples 16 to 18 were measured in accordance with the molybdenum blue method described in Example 7. As a result, when ATP and ADP, or ATP and AMP were added, the yield of the pyrophosphoric acid increased compared to the case that only ATP was added, as shown in Figure 5. This result indicated that ADP and AMP were effective as nucleotides used for the AARS reaction.

### (Comparison between yields of pyrophosphoric acids in AARS reactions described in the present invention and a known document)

### [Comparative Example 1]

In accordance with the reaction conditions described in Non-Patent Documents 6, 150 µL of a reaction solution containing 4.7 µM HisRS (derived from thermophile), 50 µM L-histidine, 0.2 mM ATP, 5 mM MgCl₂, 15 mM HEPES-KOH (pH 8) and 10 mM KCl was prepared, and subjected to enzyme reaction at 80°C for 30 minutes. After the enzyme reaction, 30 µL of trichloroacetic acid was added so that its final concentration was 4%, to terminate the reaction. After the termination of the reaction, the precipitate was removed by centrifugation to prepare Comparative Product 1.

### [Comparative Example 2]

In accordance with the reaction conditions described in Non-Patent Documents 7, 150 µL of a reaction solution containing 3.1 µM SerRS (derived from thermophile), 50 µM L-serine, 2 mM ATP, 5 mM MgCl₂, 100 mM Tris-HCl (pH 8) and 10 mM KCl was prepared, and subjected to enzyme reaction at 80°C for 30 minutes. After the enzyme reaction, 30 µL of trichloroacetic acid was added so that its final concentration was 4%, to terminate the reaction. After the termination of the reaction, the precipitate was removed by centrifugation to prepare Comparative Product 2.

### [Comparative Example 3]

In accordance with the reaction conditions described in Non-Patent Documents 6, 150 µL of a reaction solution containing 4.5 µM LysRS (derived from thermophile), 50 µM L-lysine, 0.2 mM ATP, 5 mM MgCl₂, 15 mM HEPES-KOH (pH 8) and 10 mM KCl was prepared, and subjected to enzyme reaction at 80°C for 30 minutes. After the enzyme reaction, 30 µL of trichloroacetic acid was added so that its final concentration was 4%, to terminate the reaction. After the termination of the reaction, the precipitate was removed by centrifugation to prepare Comparative Product 3.

### [Example 20]

150 µL of a reaction solution containing 5.2 µM HisRS (derived from thermophile), 50 µM L-histidine, 25.9 mM ATP, 259 mM MgCl₂ and 20 mM HEPES-KOH (pH 8) was prepared, and subjected to enzyme reaction at 70°C for 30 minutes. After the enzyme reaction, 30 µL of trichloroacetic acid was added so that its final concentration was 4%, to terminate the reaction. After the termination of the reaction, the precipitate was removed by centrifugation to prepare Example Product 34.

### [Example 21]

150 µL of a reaction solution containing 5.1 µM SerRS (derived from thermophile), 50 µM L-serine, 25.6 mM ATP, 256 mM MgCl₂ and 20 mM HEPES-KOH (pH 8) was prepared, and subjected to enzyme reaction at 70°C for 30 minutes. After the enzyme reaction, 30 µL of trichloroacetic acid was added so that its final concentration was 4%, to terminate the reaction. After the termination of the reaction, the precipitate was removed by centrifugation to prepare Example Product 35.

### [Example 22]

150 µL of a reaction solution containing 4.4 µM LysRS (derived from thermophile), 50 µM L-lysine, 22 mM ATP, 220 mM MgCl₂ and 20 mM HEPES-KOH (pH 8) was prepared, and subjected to enzyme reaction at 70°C for 30 minutes. After the enzyme reaction, 30 µL of trichloroacetic acid was added so that its final concentration was 4%, to terminate the reaction. After the termination of the reaction, the precipitate was removed by centrifugation to prepare Example Product 36.

### [Example 23]

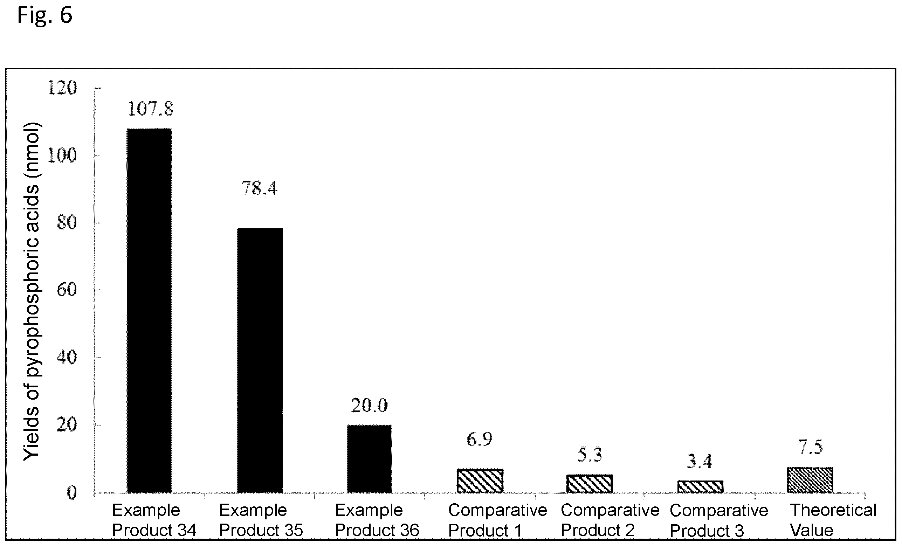

Pyrophosphoric acids of Comparative Products 1 to 3 and Example Products 34 to 36 obtained in Comparative Examples 1 to 3 and Examples 20 to 22 were measured by the molybdenum blue method described in Example 7. As a result, as shown in Figure 6, the pyrophosphoric acid was produced in an amount larger than the theoretical value of the pyrophosphoric acid presumably produced when all of the added amino acids were used in the reaction, in the method of the present invention. On the other hand, amounts of the pyrophosphoric acid for Comparative Products were the theoretical value or lower. This result indicated that the molecular number of the pyrophosphoric acid produced in the conventional measurement method using AARS was smaller than the molecular number of the amino acid, but in the method of the present invention, the pyrophosphoric acid was produced in an amount larger than the molecular number of the amino acid.

### (Comparison between yields of pyrophosphoric acids by AARS reactions with and without addition of nucleophilic agent)

### [Comparative Example 4]

150 µL of a reaction solution containing 1 µM HisRS (derived from thermophile), 30 µM L-histidine, 2 mM ATP, 20 mM MgCl₂, 400 mM hydroxylamine (nucleophilic agent) and 200 mM HEPES-KOH (pH 8) was prepared, and subjected to enzyme reaction at 70°C for 30 minutes. After the enzyme reaction, 30 µL of trichloroacetic acid was added so that its final concentration was 4%, to terminate the reaction. After the termination of the reaction, the precipitate was removed by centrifugation to prepare Comparative Product 4.

### [Example 24]

150 µL of a reaction solution containing 1 µM HisRS (derived from thermophile), 30 µM L-histidine, 2 mM ATP, 20 mM MgCl₂ and 200 mM HEPES-KOH (pH 8), and a 150 µL of reaction solution containing 5 µM SerRS (derived from thermophile), 30 µM L-serine, 2 mM ATP, 6 mM MgCl₂ and 200 mM HEPES-KOH (pH 8) were prepared, and subjected to enzyme reaction at 70°C for 30 minutes. After the enzyme reaction, 30 µL of trichloroacetic acid was added so that its final concentration was 4%, to terminate the reaction. After the termination of the reaction, the precipitate was removed by centrifugation to prepare Example Products 37 and 38.

### [Example 25]

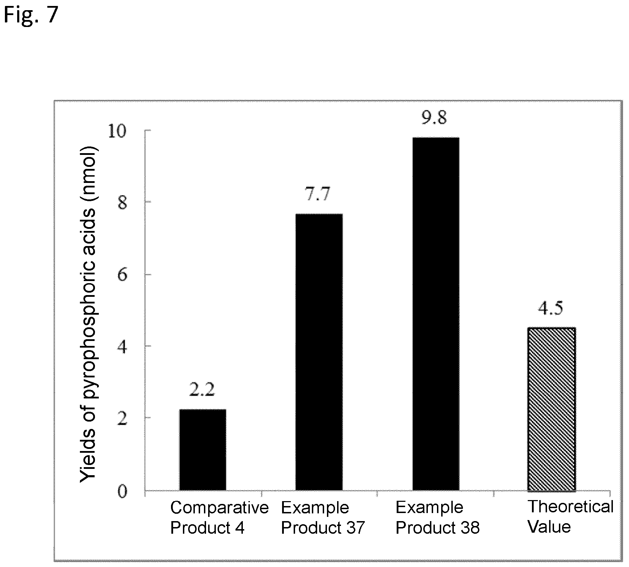

Pyrophosphoric acids of Comparative Product 4 and Example Products 37 and 38 obtained in Comparative Example 4 and Example 24 were measured by the molybdenum blue method described in Example 7. As a result, as shown in Figure 7, the pyrophosphoric acid was produced in an amount larger than the theoretical value of the pyrophosphoric acid presumably produced when all of the added amino acids were used in the reaction, in the method of the present invention. On the other hand, amounts of the pyrophosphoric acid for Comparative Products were the theoretical value or lower. This result indicated that the molecular number of the produced pyrophosphoric acid in the conventional measurement method using the AARS with the nucleophilic agent was smaller than the molecular number of the amino acid, but in the method of the present invention, the pyrophosphoric acid was produced in an amount larger than the molecular number of the amino acid.

### (Temperature dependency of AARS)

### [Example 26]

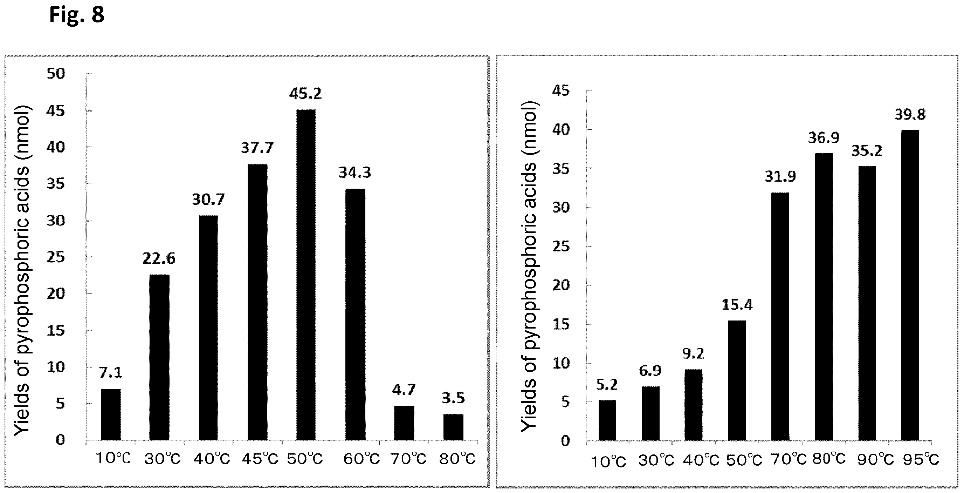

To 120 µL of a reaction solution containing 250 mM HEPES-KOH (pH 8), 31.3 mM ATP and 313 mM MgCl₂, 15 µL of L-serine was added so that its final concentration was 50 µM, and 15 µL of SerRS (derived from E. coli) was added so that its final concentration was 5 µM to prepare an enzyme reaction solution, which was reacted at 10°C, 30°C, 40°C, 45°C, 50°C, 60°C, 70°C and 80°C respectively for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 8, it was found that the pyrophosphoric acid was produced within a temperature range of 10 to 80°C, particularly pyrophosphoric acid was well produced within a temperature range of 30 to 60°C.

### [Example 27]

To 120 µL of a reaction solution containing 250 mM HEPES-KOH (pH 8), 12.5 mM ATP and 125 mM MgCl₂, 15 µL of L-histidine was added so that its final concentration was 30 µM, and 15 µL of HisRS (derived from thermophile) was added so that its final concentration was 5 µM to prepare an enzyme reaction solution, which was reacted at 10°C, 30°C, 40°C, 50°C, 70°C, 80°C, 90°C and 95°C respectively for 15 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 8, it was found that the pyrophosphoric acid was well produced within a temperature range of 10 to 95°C.

### (Preparation of calibration curve for amino acid in pyrophosphoric acid measurement in accordance with molybdenum blue method)

### [Example 28]

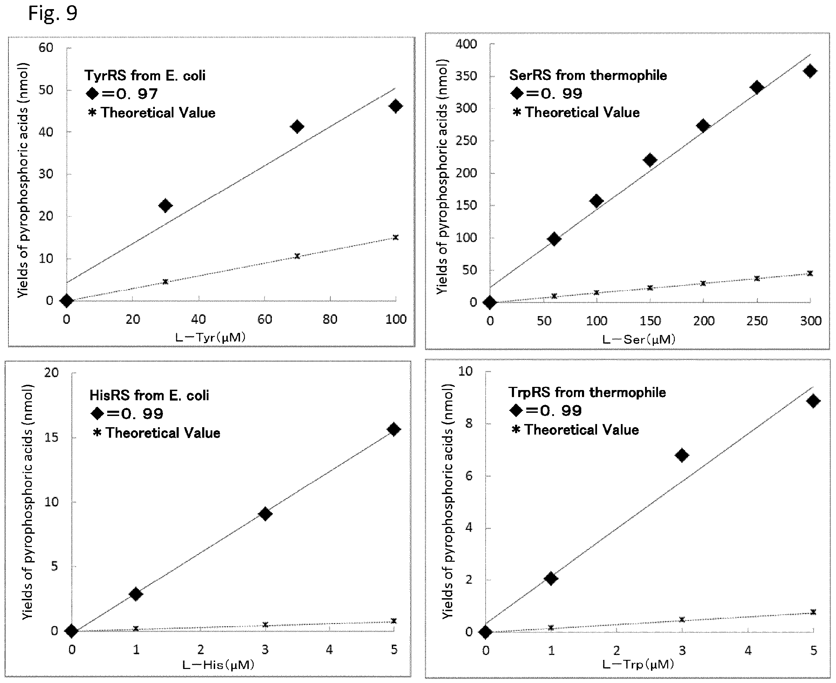

To 120 µL of a reaction solution containing 250 mM HEPES-KOH (pH 8), 31.3 mM ATP and 313 mM MgCl₂, 15 µL of L-tyrosine was added so that its final concentration was 0 µM, 30 µM, 70µM or 100µM to prepare each sample, then to each sample, 15 µL of TyrRS (derived from E. coli) was further added so that its final concentration was 5µM, and the sample was reacted at 50°C for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 9, the pyrophosphoric acid was produced in an amount larger than the theoretical value of the pyrophosphoric acid presumably produced when all of the added amino acids were used in the reaction. In addition, a correlation between the amino acid concentration and the pyrophosphoric acid amount (R=0.97) was found in a range of the amino acid concentration of 0 to 100 µM, indicating that quantification of L-tyrosine was possible.

### [Example 29]

To 120 µL of a reaction solution containing 250 mM HEPES-KOH (pH 8), 31.3 mM ATP and 313 mM MgCl₂, 15 µL of L-serine was added so that its final concentration was 0 µM, 60 µM, 100 µM, 150 µM, 200 µM, 250 µM or 300µM to prepare each sample, then to each sample, 15 µL of SerRS (derived from thermophile) was further added so that its final concentration was 5µM, and the sample was reacted at 70°C for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 9, the pyrophosphoric acid was produced in an amount larger than the theoretical value of the pyrophosphoric acid presumably produced when all of the added amino acids were used in the reaction. In addition, a correlation between the amino acid concentration and the pyrophosphoric acid amount (R=0.99) was found in a range of the amino acid concentration of 0 to 300 µM, indicating that quantification of L-serine was possible.

### [Example 30]

To 120 µL of a reaction solution containing 250 mM HEPES-KOH (pH 8), 31.3 mM ATP and 313 mM MgCl₂, 15 µL of L-histidine was added so that its final concentration was 0 µM, 1.0 µM, 3.0 µM or 5.0 µM to prepare each sample, then to each sample, 15 µL of HisRS (derived from E. coli) was further added so that its final concentration was 5µM, and the sample was reacted at 50°C for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 9, the pyrophosphoric acid was produced in an amount larger than the theoretical value of the pyrophosphoric acid presumably produced when all of the added amino acids were used in the reaction. In addition, a correlation between the amino acid concentration and the pyrophosphoric acid amount (R=0.99) was found in a range of the amino acid concentration of 0 to 5 µM, indicating that quantification of L-histidine was possible.

### [Example 31]

To 120 µL of a reaction solution containing 250 mM HEPES-KOH (pH 8), 31.3 mM ATP and 313 mM MgCl₂, 15 µL of L-tryptophan was added so that its final concentration was 0 µM, 1.0 µM, 3.0 µM or 5.0 µM to prepare each sample, then to each sample, 15 µL of TrpRS (derived from thermophile) was further added so that its final concentration was 5µM, and the sample was reacted at 70°C for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 9, the pyrophosphoric acid was produced in an amount larger than the theoretical value of the pyrophosphoric acid presumably produced when all of the added amino acids were used in the reaction. In addition, a correlation between the amino acid concentration and the pyrophosphoric acid amount (R=0.99) was found in a range of the amino acid concentration of 0 to 5 µM, indicating that quantification of L-tryptophan was possible.

### (Preparation of calibration curve for amino acid in hydrogen ion concentration measurement by cumulative ISFET electrode)

### [Example 32]

100 µL of samples respectively containing TrpRS (derived from thermophile) at a final concentration of 5 µM, L-tryptophan at a final concentration of 0 µM, 15 µM, 20 µM, 40 µM or 50 µM, ATP at a final concentration of 10 mM, MgCl₂ at a final concentration of 100 mM and HEPES-KOH (pH 8) at a final concentration of 1 mM as reaction compositions were prepared, and reacted at 70°C for 30 minutes. After the reaction, they were allowed to stand at room temperature for 10 minutes.

### [Example 33]

100 µL of samples respectively containing LysRS (derived from thermophile) at a final concentration of 5 µM, L-lysine at a final concentration of 0 µM, 15 µM, 20 µM, 40 µM or 50 µM, ATP at a final concentration of 10 mM, MgCl₂ at a final concentration of 100 mM and HEPES-KOH (pH 8) at a final concentration of 1 mM as reaction compositions were prepared, and treated in the same manner as in Example 32.

### [Example 34]

100 µL of each sample containing SerRS (derived from thermophile) at a final concentration of 5 µM, L-serine at a final concentration of 0 µM, 20 µM, 50 µM or 60 µM, ATP at a final concentration of 10 mM, MgCl₂ at a final concentration of 100 mM and HEPES-KOH (pH 8) at a final concentration of 1 mM as reaction compositions was prepared, and treated in the same manner as in Example 32.

### [Example 35]

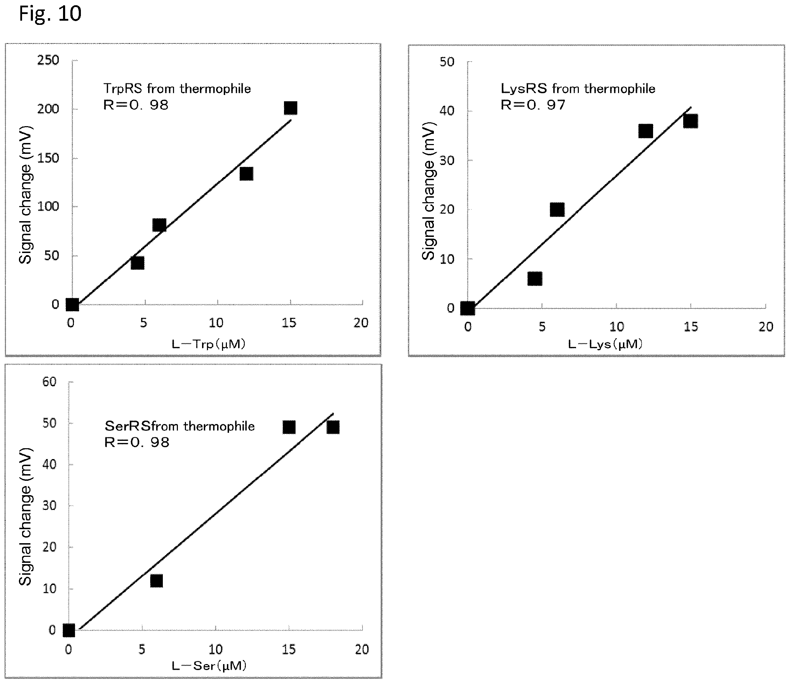

Measurement using a physiological activity reaction measurement apparatus (AMIS-101X, made by Bio-X Inc.) was carried out. As a cumulative ISFET sensor, a reference electrode-housing AMIS sensor (AMIS-051) was used. 70 µL of a solution containing HEPES-KOH (pH 8) at a final concentration of 1 mM, MgCl₂ at a final concentration of 200 mM and KCl at a final concentration of 10 mM was added to each of sensing parts A and B in the AMIS-051. Preheating was carried out at 30°C for 3 minutes, and after the signal was stabilized, 30 µL of the prepared measurement object (each sample in Examples 32 to 34) was added to the sensing part A and 30 µL of a solution which had the same composition as of each sample in Examples 32 to 34 except that water was added instead of the amino acid was added to the sensing part B, and mixed to measure an amount of signal change (signal change at the sensing part A compared to the signal at the sensing part B) every 5 seconds for 250 seconds. The cumulative frequency of the cumulative ISFET sensor was 10 for this measurement. As a result, as shown in Figure 10, a calibration curve can be prepared for each amino acid, indicating that the amino acids could be quantified in the cumulative ISFET sensor.

### (Yield of pyrophosphoric acid depending on various ATP concentrations and divalent ions in various AARSs using D-amino acids: Step (I) in the method of the present invention)

### [Example 36]

240 µL of a reaction solution containing 250 mM HEPES-KOH (pH 8), 31.3 mM ATP and 31.3 mM MnCl₂, or 240 µL of a reaction solution containing 250 mM HEPES-KOH (pH 8), 50 mM ATP and 50 mM MnCl₂, or 240 µL of a reaction solution containing 250 mM HEPES-KOH (pH 8), 62.5 mM ATP and 62.5 mM MnCl₂ was prepared, and to each solution, 30 µL of D-histidine was added so that its final concentration was 50 µM, and 30 µL of HisRS (derived from E. coli) was added so that its final concentration was 5 µM, and the solution was treated at 50°C for 30 minutes. After the reaction, 60 µL of trichloroacetic acid was added so that its final concentration was 4%, to terminate the reaction. After the termination of the reaction, the precipitate was removed by centrifugation to prepare Example Products 39 to 41.

### [Example 37]

240 µL of a reaction solution containing 250 mM HEPES-KOH (pH 8), 31.3 mM ATP and 31.3 mM MnCl₂, or 240 µL of a reaction solution containing 250 mM HEPES-KOH (pH 8), 50 mM ATP and 50 mM MnCl₂, or 240 µL of a reaction solution containing 250 mM HEPES-KOH (pH 8), 62.5 mM ATP and 62.5 mM MnCl₂ were prepared, and to each solution, 30 µL of D-tryptophan was added so that its final concentration was 50 µM, and 30 µL of TrpRS (derived from thermophile) was added so that its final concentration was 5 µM, and the solution was treated at 70°C for 30 minutes. After the reaction, 60 µL of trichloroacetic acid was added so that its final concentration was 4%, to terminate the reaction. After the termination of the reaction, the precipitate was removed by centrifugation to prepare Example Products 42 to 44.

### (Measurement of pyrophosphoric acid in accordance with molybdenum blue method: Step (II) in the method of the present invention)

### [Example 38]

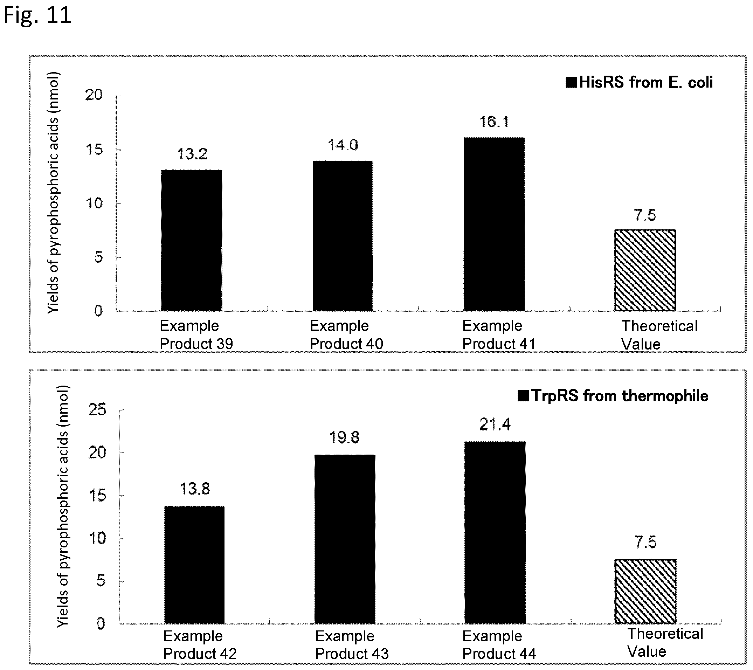

As a result of measuring Example Products 39 to 44 prepared in Examples 36 and 37 in accordance with the molybdenum blue method described in Examples 7, it was found that the pyrophosphoric acid increased as the concentrations of ATP and MnCl₂ increased, as shown in Figure 11. In addition, the pyrophosphoric acid was produced in an amount larger than the theoretical value of the pyrophosphoric acid produced when all of the added amino acids were used in the enzyme reaction. Thus, it was shown that the pyrophosphoric acid was produced in a molar number larger than the molecular number of the amino acids contained in the sample in accordance with the method of the present invention.

### (Preparation of calibration curve for D-amino acid in pyrophosphoric acid measurement in accordance with molybdenum blue method)

### [Example 39]

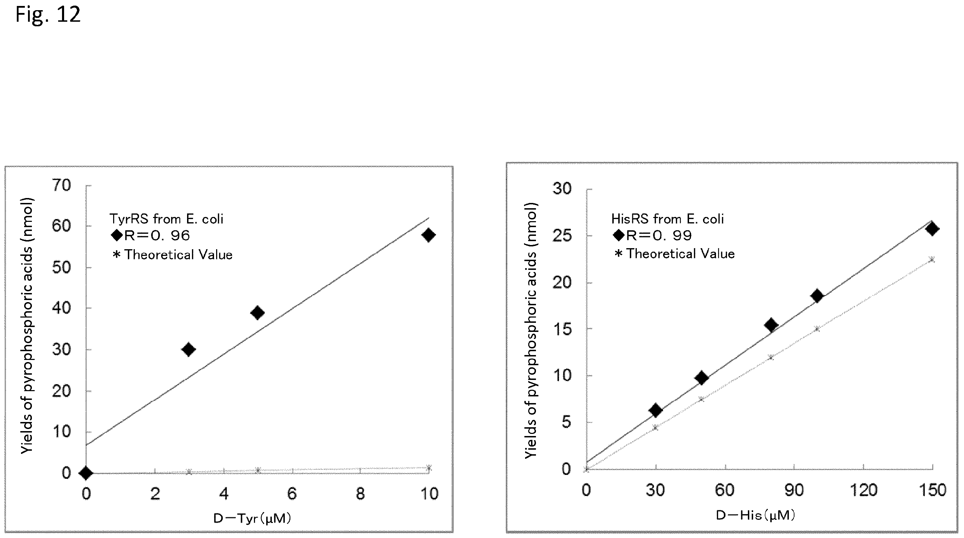

To 120 µL of a reaction solution containing 250 mM HEPES-KOH (pH 8), 50 mM ATP and 50 mM MnCl₂, 15 µL of D-tyrosine was added so that its final concentration was 0 µM, 3 µM, 5 µM or 10µM to prepare each sample, then to each sample, 15 µL of TyrRS (derived from E. coli) was further added so that its final concentration was 5 µM, and the sample was reacted at 40°C for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 12, the pyrophosphoric acid was produced in an amount larger than the theoretical value of the pyrophosphoric acid presumably produced when all of the added amino acids were used in the reaction. In addition, a correlation between the amino acid concentration and the pyrophosphoric acid amount (R=0.96) was found in a range of the amino acid concentration of 0 to 10 µM, indicating that quantification of D-tyrosine was possible.

### [Example 40]

To 120 µL of a reaction solution containing 250 mM HEPES-KOH (pH 8), 50 mM ATP and 50 mM MnCl₂, 15 µL of D-histidine was added so that its final concentration was 0 µM, 30 µM, 50 µM, 80 µM, 100 µM or 150 µM to prepare each sample, then to each sample, 15 µL of HisRS (derived from E. coli) was further added so that its final concentration was 5 µM, and the sample was reacted at 40°C for 30 minutes. After the reaction, 30 µL of trichloroacetic acid was added so that its final concentration was 4%, to terminate the reaction. After the termination of the reaction, the precipitate was removed by centrifugation, and the pyrophosphoric acid in the supernatant was measured in accordance with the molybdenum blue method described in Example 7. As a result, as shown in Figure 12, the pyrophosphoric acid was produced in an amount larger than the theoretical value of the pyrophosphoric acid presumably produced when all of the added amino acids were used in the reaction. In addition, a correlation between the amino acid concentration and the pyrophosphoric acid amount (R=0.99) was found in a range of the amino acid concentration of 0 to 150 µM, indicating that quantification of D-histidine was possible.

### (Measurement of D-amino acid after removal of L-amino acid)

### [Example 41]

A solution was prepared so as to contain HEPES-KOH (pH 8) at a final concentration of 200 mM, L-tryptophan at a final concentration of 0.5 mM, D-tryptophan at a final concentration of 0.5 mM, pyridoxal phosphate at a final concentration of 25 µM and tryptophanase at a final concentration of 0.8 U/mL, and reacted at 37°C for 5 minutes. After the reaction, the solution was subjected to heat treatment at 80°C for 30 minutes, and the precipitate was removed by centrifugation.

### [Example 42]

30 µL of the amino acid solution prepared in Example 41 was added to 240 µL of a reaction solution containing 250 mM HEPES-KOH (pH 8), 50 mM ATP and 50 mM MnCl₂ to prepare each sample, then to each sample, 30 µL of TrpRS (derived from E. coli) was further added so that its final concentration was 5 µM, and the sample was reacted at 40°C for 30 minutes. After the reaction, 60 µL of trichloroacetic acid was added so that its final concentration was 4%, to terminate the reaction. After the termination of the reaction, the precipitate was removed by centrifugation to prepare Example Product 45. In the same manner, Comparative Products 5 and 6 were prepared in order to measure the yield of the pyrophosphoric acid produced by the AARS reaction from an amino acid solution containing only 0.5 mM D-tryptophan or 0.5 mM L-tryptophan as amino acids and not treated with tryptophanase.

### [Example 43]

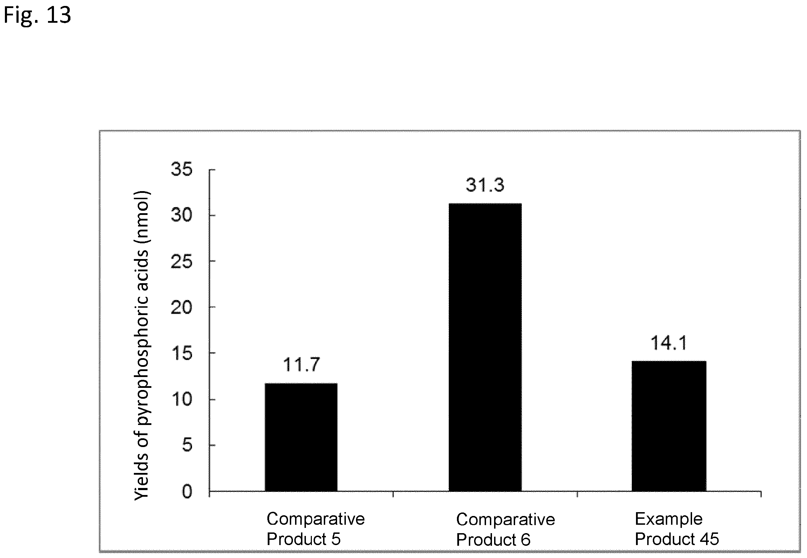

Pyrophosphoric acids in supernatants of Example Product 45 and Comparative Products 5 and 6 obtained in Example 42 were measured in accordance with the molybdenum blue method described in Example 7. As a result, as shown in Figure 13, Example Product 45 and Comparative Product 5 showed substantially equivalent yields of pyrophosphoric acid. From this result, it was considered that the L-tryptophan in the mixture solution of D-form and L-form tryptophans could been removed by enzyme treatment. Thus, it was indicated that the D-amino acid in the mixture solution of D-form and L-form amino acids could be measured by removing the L-amino acid.

The above results indicated that it was difficult to produce pyrophosphoric acid produced by the AARS reaction in an amount larger than the molar number of the amino acids contained in the sample in the conventional method described in the known documents as shown in Comparative Examples 1 to 4, but in the method of the present invention, reaction products could be amplified by repeatedly using the AARS and the amino acids for reactions. From this fact, in the AARS reaction in the method of the present invention, the pyrophosphoric acid could be produced in the reaction in an amount larger than the molar numbers of amino acids in both cases of the L-form and D-form, as shown in Examples 3 to 19 and Examples 36 to 38. In addition, it was found that the yield of the pyrophosphoric acid varied depending on the enzyme reaction temperature, and the AARS reaction preferably occurred at 10 to 95°C for the AARSs derived from E. coli and thermophile, as shown in Examples 26 and 27. Furthermore, as shown in Examples 28 to 31 and Examples 39 and 40, it was found that the pyrophosphoric acid linearly increased depending on the amino acid concentration for each AARS in both cases of L-form and D-form amino acids, i.e. there was a correlation between the amino acid concentration and the pyrophosphoric acid amount, and it was confirmed that calibration curves of various amino acids in accordance with the molybdenum blue method that is a simple method could be prepared for the pyrophosphoric acid produced by the AARS reaction of the present invention. In addition, from Examples 41 to 43, it was confirmed that, in the case of the mixture solution of the L-form and D-form amino acids, after removing one amino acid, the remaining amino acid could be measured by AARS.

As apparent from the above description, even when using a simple method such as the molybdenum blue method, the method of the present invention allows quantification of the amino acids in a concentration range of 1 to 300 µM, and this range was comparable to the amino acid quantification range of 1 to 250 µM in the amino acid-quantifying method of high sensitivity analysis in the prior art. In addition, as shown in Example 35, calibration curves for various amino acids could be prepared by the cumulative ISFET electrode. The amino acid quantification range was 0 to 20 µM, and thus it was found that the amino acids could be quantified in a concentration range significantly lower than the amino acid quantification range of 300 to 900 µM for the ISFET electrode of the prior art (90 to 270 µM when converted to the range for the cumulative ISFET electrode used in the above Examples).

### Industrial Applicability

As described above, in the conventional amino acid quantifying method using the AARS, the amounts of the produced pyrophosphoric acid and the like were small, and thus a high-sensitivity analysis in accordance with fluorometry or the like using a multistep enzyme reaction was necessary. However, in the method of the present invention, even when only a small amount of amino acid was contained in a sample, a large amount of pyrophosphoric acid and hydrogen ions could be produced by releasing the AARS and amino acids from the formed aminoacyl AMP-AARS complex and repeatedly reusing them for formation of the aminoacyl AMP-AARS complex, and thus it was found that the high-sensitivity analysis by means of fluorometry or the like using the multistep enzyme reaction was unnecessary. Consequently, according to the present invention, it became possible to provide a method for selectively and easily quantifying amino acids to be measured with high sensitivity using an AARS.

## Claims

1. A method for quantifying amino acids in a sample, comprising:
Step (I) comprising following steps:
(Step 1-1) a step including a reaction (Reaction 1) wherein L-form and/or D-form amino acids (L-AA and/or D-AA) in the sample, an aminoacyl tRNA synthetase (AARS) corresponding to the amino acids and an adenosine triphosphate (ATP) are reacted in the presence of a divalent ion or a polyamine to form a complex comprising an aminoacyl adenylate (aminoacyl AMP) and the AARS (aminoacyl AMP-AARS complex);
(Step I-2) a step including a reaction (Reaction 2) wherein the aminoacyl AMP-AARS complex formed in Reaction 1 and/or Reaction 3 is reacted with a nucleotide to release the AARS and the amino acids (L-AA and/or D-AA) from the complex;
(Step I-3) a step including a reaction (Reaction 3) wherein the amino acids (L-AA and/or D-AA) and/or the AARS released in Reaction 2 are reused in Reaction 1 to cause the aminoacyl AMP-AARS complex reaction; and
(Step I-4) a step of repeating the Step I-2 and the Step I-3, and
Step (II) comprising measuring an amount of reaction products produced in the Step (I), wherein the molar number of the reaction products produced in Step (I) is larger than that of the amino acids in the sample, and determining an amount of the L-form and/or D-form amino acids on the basis of the measured amount of the reaction products.

2. The method for quantifying amino acids according to claim 1, wherein the amount of the reaction products produced in the Step (I) is measured by measuring potential change by an ion-sensitive field effect transistor, a glass electrode membrane or a multielectrode electrometer.

3. The method for quantifying amino acids according to claim 1, wherein the amount of the reaction products produced in the Step (I) is measured by measuring change in absorbance in accordance with absorptiometry.

4. The method for quantifying amino acids according to any one of claims 1 to 3, wherein at least one of a pyrophosphoric acid and hydrogen ion is measured as the reaction products produced in the Step (I).

5. The method for quantifying amino acids according to any one of claims 1 to 4, further comprising a pretreatment step for removing either one of the L-form and D-form amino acids in the sample.

## Patentansprüche

1. Verfahren zum Quantifizieren von Aminosäuren in einer Probe, umfassend:
Schritt (I), umfassend die folgenden Schritte:
(Schritt 1-1) einen Schritt, beinhaltend eine Reaktion (Reaktion 1), wobei L-Form- und/oder D-Form-Aminosäuren (L-AA und/oder D-AA) in der Probe, eine Aminoacyl-tRNA-Synthetase (AARS), die den Aminosäuren entspricht, und ein Adenosintriphosphat (ATP) in Gegenwart eines zweiwertigen Ions oder eines Polyamins umgesetzt werden, um einen Komplex zu bilden, umfassend ein Aminoacyladenylat (Aminoacyl-AMP) und die AARS (Aminoacyl-AMP-AARS-Komplex);
(Schritt 1-2) einen Schritt, beinhaltend eine Reaktion (Reaktion 2), wobei der in Reaktion 1 und/oder Reaktion 3 gebildete AminoacylAMP-AARS-Komplex mit einem Nukleotid umgesetzt wird, um die AARS und die Aminosäuren (L-AA und/oder D-AA) aus dem Komplex freizusetzen;
(Schritt 1-3) einen Schritt, beinhaltend eine Reaktion (Reaktion 3), wobei die Aminosäuren (L-AA und/oder D-AA) und/oder die in Reaktion 2 freigesetzte AARS in Reaktion 1 wiederverwendet werden, um die AminoacylAMP-AARS-Komplex-Reaktion auszulösen; und
(Schritt 1-4) einen Schritt des Wiederholens des Schritts 1-2 und des Schritts 1-3 und
Schritt (II), umfassend Messen einer Menge an in Schritt (I) produzierten Reaktionsprodukten, wobei die in Schritt (I) produzierte molare Anzahl der Reaktionsprodukte größer ist als die der Aminosäuren in der Probe, und Bestimmen einer Menge der L-Form- und/oder D-Form-Aminosäuren auf der Grundlage der gemessenen Menge der Reaktionsprodukte.

2. Verfahren zum Quantifizieren von Aminosäuren nach Anspruch 1, wobei die Menge der in Schritt (I) produzierten Reaktionsprodukte durch Messen von Potentialänderung durch einen ionenempfindlichen Feldeffekttransistor, eine Glaselektrodenmembran oder ein Multielektrodenelektrometer gemessen wird.

3. Verfahren zum Quantifizieren von Aminosäuren nach Anspruch 1, wobei die Menge der in Schritt (I) produzierten Reaktionsprodukte durch Messen von Änderung in Absorbanz in Übereinstimmung mit Absorptiometrie gemessen wird.

4. Verfahren zum Quantifizieren von Aminosäuren nach einem der Ansprüche 1 bis 3, wobei mindestens eins von einer Pyrophosphorsäure und einem Wasserstoffion als die in Schritt (I) produzierten Reaktionsprodukte gemessen wird.

5. Verfahren zum Quantifizieren von Aminosäuren nach einem der Ansprüche 1 bis 4, weiter umfassend einen Vorbehandlungsschritt zum Entfernen einem von entweder der L-Form- oder D-Form-Aminosäuren in der Probe.

## Revendications

1. Procédé de quantification d'acides aminés dans un échantillon, comprenant :
une étape (I) comprenant les étapes suivantes :
(étape I-1) une étape incluant une réaction (réaction 1) dans laquelle des acides aminés de forme L et/ou de forme D (L-AA et/ou D-AA) dans l'échantillon, une aminoacyl-ARNt synthétase (AARS) correspondant aux acides aminés et un triphosphate d'adénosine (ATP) sont mis en réaction en présence d'un ion divalent ou d'une polyamine pour former un complexe comprenant un aminoacyl-adénylate (aminoacyl-AMP) et l'AARS (complexe aminoacyl-AMP-AARS) ;
(étape I-2) une étape incluant une réaction (réaction 2) dans laquelle le complexe aminoacyl-AMP-AARS formé dans la Réaction 1 et/ou la Réaction 3 est mis en réaction avec un nucléotide pour libérer l'AARS et les acides aminés (L-AA et/ou D-AA) du complexe ;
(étape I-3) une étape incluant une réaction (réaction 3) dans laquelle les acides aminés (L-AA et/ou D-AA) et/ou l'AARS libérés dans la Réaction 2 sont réutilisés dans la Réaction 1 pour provoquer la réaction du complexe aminoacyl-AMP-AARS ; et
(étape I-4) une étape consistant à répéter l'étape I-2 et l'étape I-3, et
une étape (II) comprenant la mesure d'une quantité de produits de réaction produits à l'étape (I), dans laquelle le nombre molaire des produits de réaction produits à l'étape (I) est supérieur à celui des acides aminés dans l'échantillon, et la détermination d'une quantité d'acides aminés de forme L et/ou de forme D sur la base de la quantité mesurée des produits de réaction.

2. Procédé de quantification d'acides aminés selon la revendication 1, dans lequel la quantité de produits de réaction produits à l'étape (I) est mesurée en mesurant une modification de potentiel par un transistor à effet de champ sensible aux ions, une membrane d'électrode de verre ou un électromètre à électrodes multiples.

3. Procédé de quantification d'acides aminés selon la revendication 1, dans lequel la quantité de produits de réaction produits à l'étape (I) est mesurée en mesurant une modification de l'absorbance selon un processus d'absorptiométrie.

4. Procédé de quantification d'acides aminés selon l'une quelconque des revendications 1 à 3, dans lequel au moins un d'un acide pyrophosphorique et d'un ion hydrogène est mesuré au titre des produits de réaction produits à l'étape (I).

5. Procédé de quantification d'acides aminés selon l'une quelconque des revendications 1 à 4, comprenant en outre une étape de prétraitement pour extraire l'un ou l'autre des acides aminés de forme L et de forme D dans l'échantillon.
